# EUROPÄISCHE PATENTSCHRIFT

(11) **EP 0 412 280 B1**
(45) Veröffentlichungstag und Bekanntmachung des Hinweises auf die Patenterteilung: **16.03.1994**
(21) Anmeldenummer: 90112387.7
(22) Anmeldetag: 29.06.1990
(51) Int. Cl.: A61B 17/58

(54) **Befestigungsmittel**
Fastening means
Moyen de fixation

(30) Priorität: 08.08.1989 DE 3926127
(43) Veröffentlichungstag der Anmeldung: 13.02.1991
(73) Patentinhaber: BIOVISION GmbH Entwicklung, Herstellung und Vertrieb von Biomaterialien, D-98693 Ilmenau (DE)
(72) Erfinder: Claes, Lutz, Prof. Dr., D-7910 Neu-Ulm/Pfuhl (DE); Rehm, Klaus, Prof. Dr., D-5030 Hürth-Efferen (DE)
(74) Vertreter: Rackette, Karl, Dipl.-Phys. Dr.-Ing

(56) Entgegenhaltungen:
- EP-A- 360 139
- EP-A- 0 146 699
- EP-A- 0 260 222
- FR-A- 2 357 229

## Beschreibung

Die Erfindung betrifft ein Befestigungsmittel zur Fixierung von Knochenfragmenten mit mindestens einem mit einem röntgenologisch darstellbaren Kontrastmittel gefüllten Aufnahmeraum.

Die CH 661 199 A5 beschreibt das Einbringen eines Kontrastkörpers in Gestalt einer Kugel in ein für Röntgenstrahlen durchsichtiges Implantat, um einen röntgenologischen Bezus- und Markierungspunkt zu schaffen. Die Markierungskugel ist insbesondere korrosionsfest oder durch eine Schutzflüssigkeit gegen Auflösung geschützt.

In der DE-OS 27 30 571 ist die Verwendung von Bariumsulfat als röntgenologisches Kontrastmittel beschrieben.

Aus der DE-OS 22 46 940 ist bekannt. Stahldrähte als Markierungsdrähte tangential an der Oberfläche des Befestigungsmittels anzuordnen.

Die DE 88 15 977 U1 beschreibt einen Knochennagel mit mehreren umfänglichen Rippen.

Aus der EP-OS 0 276 153 ist eine Knochenschraube aus resorbierbarem Kunststoff bekannt. Knochenschrauben aus bioresorbierbarem Material weisen den Vorteil auf, daß sie während des Heilungsprozesses vom Körper resorbiert werden, so daß eine zusätzliche Operation zur Entfernung derartiger Implantate überflüssig wird.

In der DE-GM 88 04 456 ist ebenfalls eine andere Knochenschraube aus resorbierbarem Kunststoffmaterial beschrieben.

EP-A-360 139, ein Dokument, das unter Art. 54(3) EPü fällt, beschreibt ein Befestigungsmittel aus bioresorbierbarem Werkstoff, bei dem in die Spitze der Gewindestange bzw. der Schraube eine Calciumphosphatkugel eingepreßt ist, um die Lage des Befestigungsmittels durch Röntgen nach zu prüfen. Bei Knochenplatten wird das Calciumphosphat in großflächiger Weise auf die Befestigungsplatten aufgebracht, so daß durch das röntgenologisch nachweisbare Calciumphosphat die Lage der Befestigungsplatte im Körper eines Patienten beurteilt werden kann.

Aus dem Firmenkatalog Ethipin der Firma Ethicon GmbH, 2000 Norderstedt, sind Knochenstifte aus resorbierbarem Material bekannt. Diese Stifte weisen den Nachteil auf, daß sie nur Torsionsmomente aufnehmen können. Druckkräfte können mit ihnen nicht übertragen werden.

Alle vorbekannten Befestigungsmittel weisen den Nachteil auf, daß die Knochenbildung und die eventuelle fortschreitende Auflösung des Befestigungsmittels nicht überwacht werden können. Insbesondere sind bioresorbierbare Werkstoffe röntgenologisch nicht darstellbar und somit nicht lokalisierbar.

Ausgehend von diesem Stand der Technik liegt der Erfindung die Aufgabe zugrunde, ein Befestigungsmittel zu schaffen, bei dessen Einsatz der Heilungsprozeß des verletzten Knochens eines Patienten in einfacher Weise überwacht werden kann.

Diese Aufgabe wird erfindungsgemäß dadurch gelost, daß das Befestigungsmittel zur Fixierung von Knochenfragmenten mindestens einen mit einem röntgenologisch darstellbaren Kontrastmittel gefüllten Aufnahmeraum aufweist, wobei das Kontrastmittel in einer durch die Art des Kontrastmittels, dessen Konzentration, der Art des bioresorbierbaren Werkstoffs und die Außenwanddicke des Aufnahmeraums vorbestimmten Zeitdauer langsam parallel zur Auflösung des Befestigungsmittels durch die Aufnahmeraumwände hindurchdiffundierbar ist.

Dadurch ist mit Hilfe einer einfachen röntgenologischen Aufnahme des behandelten Körperteils der Fortschritt des Heilungsprozesses feststellbar, da die Diffusionsgeschwindigkeit des Kontrastmittels aus dem Befestigungsmittel in hohem Maße von der Auflösung des Befestigungsmittels bei der Bildung neuen Knochenmaterials abhängt.

Bei vorteilhaften Ausführungsbeispielen ist das Kontrastmittel in einem Knochenstift oder in einer Knochenschraube in einem zylindrischen Aufnahmeraum in der Spitze des Schaftes oder im Kopfbereich eingebracht, so daß die Auflösung des Befestigungsmittels im Knochentiefsten bzw. an seiner Oberfläche durch eine einfache Röntgenaufnahme nachweisbar ist.

Die Verwendung von pulverförmigen Bariumsulfat gestattet die Erhöhung der Standzeit des Kontrastmittels, so daß die normale Diffusion durch die Außenwände des Befestigungsmittels hindurch nicht schneller als der das Befestigungsmittel auflösende Heilungs- und Bildungsprozeß des Knochens abläuft.

Nachfolgend wird die Erfindung anhand der Zeichnung beispielhaft näher erläutert. Es zeigen:
- Fig. 1: einen Knochennagel gemäß einem ersten Ausführungsbeispiel der Erfindung,
- Fig. 2: eine Knochensschraube gemäß einem zweiten Ausführungsbeispiel der Erfindung, und
- Fig. 3: eine Knochenplatte gemäß einem dritten Ausführungsbeispiel der Erfindung.

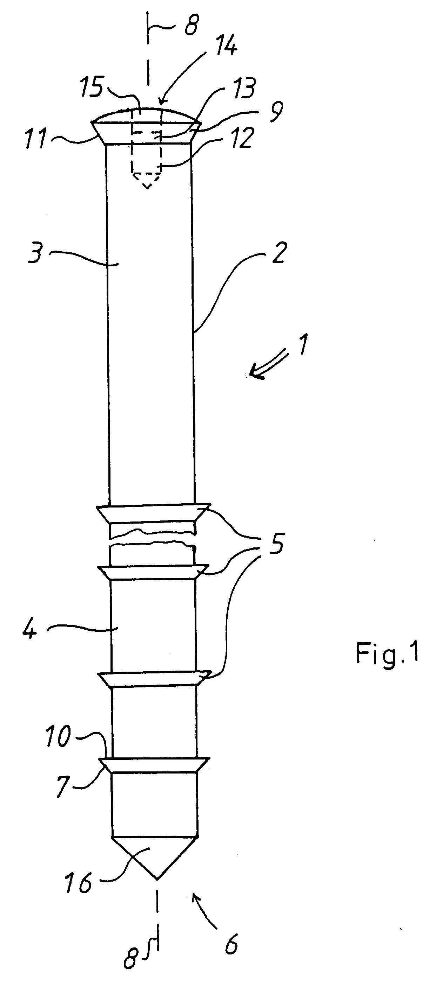

Das in der Fig. 1 dargestellte Befestigungsmittel aus bioresorbierbarem Werkstoff zur Fixierung von Knochenfragmenten besteht aus einem Knochennagel 1. Der Knochennagel 1 verfügt über einen länglichen Schaft 2, der in seinem oberen Bereich 3 glatt ist. Ein unterer Bereich 4 des Schaftes 2 ist mit Rippen 5 versehen, die umfänglich um den Schaft angeordnet sind.

Der die Form eines Stiftes aufweisende Knochennagel 1 wird bei einer Operation durch das Knochenfragment des abgesplitterten Knochens und durch das Knochen-Fragmentlager in einem Bohrkanal in die Knochensubstanz eingeführt. Die Rippen 5 wirken dabei als in das umgebende Knochengewebe eingreifende Verankerungsstufen.

Der maximale Durchmesser der Rippen 5 ist um 5 bis 25 Prozent größer als der Durchmesser des Schaftes 2. Bei einem Schaft 2 mit einem Durchmesser von 2 Millimeter beträgt eine geeignete Länge des Knochennagels 1 35 bis 40 Millimeter. Der glatte obere Bereich 3 des Schaftes 2 weist eine Länge von ungefähr 1 Zentimeter auf. Auf dem unteren Schaftabschnitt 4 sind ungefähr ein Dutzend Rippen 5 vorgesehen.

Der maximale Durchmesser der Rippen 5 bei einem Schaftdurchmesser von 2 Millimeter beträgt etwa 2,3 Millimeter. Im Querschnitt weisen die über den Schaft 2 überstehenden Rippen 5 die Form eines Sägezahns oder Dreiecks auf. Dabei bildet die dem einführtiefen Ende 6 des Knochennagels zugewandte Seite 7 des Dreiecks mit der Achse 8 des Schaftes 2 einen spitzen Winkel im Bereich zwischen 30 und 60 Grad. Die Auszugsfestigkeit des Knochennagels ist am größten, wenn der spitze Winkel einen Wert von 45 Grad aufweist.

Die dem Kopf 9 des Knochennagels zugewandte Seite 10 des oben benannten Dreiecks bildet mit der Achse 8 des Schaftes 2 einen rechten Winkel. Vorzugsweise ist dann zwischen der einführtiefen Seite 7 und der dem Kopf 9 zugewandten Seite 10 ein Winkel von 45 Grad ausgebildet.

Der Kopf 9 des Knochennagels 1 verfügt über eine konische Verbreiterung 11 des Schaftes 2, wobei der größte Durchmesser des Kopfes 9 zwischen 10 und 50 Prozent größer als der Durchmesser des Schaftes 2 ist. Bei einem Knochennagel 1 mit einem Durchmesser von 2 Millimetern ist der größte Durchmesser des Kopfes 9 zum Beispiel 2,6 Millimeter.

Der im Querschnitt zu erkennende spitze Winkel zwischen der Achse 8 des Knochennagels 1 und der konischen Verbreiterung 11 liegt im Bereich zwischen 20 und 60 Grad, vorteilhafterweise bei 35 Grad. Die rückseitige Endfläche der konischen Verbreiterung 11 des Kopfes 9 ist ballig ausgeformt.

Entlang der Achse 8 des Knochennagels 1 ist in dem Kopf 9 ein sich bis in den Schaft 2 erstreckender Aufnahmeraum 12 vorgesehen, der einen Durchmesser von zum Beispiel 0,6 Millimeter und eine Länge von 0,5 Millimeter aufweist. In das so sich ergebende Volumen des Aufnahmeraums 12 von ungefähr 0,15 Kubikmillimeter ist ein röntgenologisch darstellbares Kontrastmittel 13 eingefüllt. Das Kontrastmittel 13, zum Beispiel Bariumsulfat, kann pulverförmig oder in flüssiger Form vorgesehen sein. In flüssiger Form ist das Bariumsulfat z.B. in einem Polymer gelöst. Es kann auch eine nichtionische Injektionslösung als Lösungsmittel verwendet werden.

Die an der rückseitigen Endfläche vorhandene Öffnung 14 des Aufnahmeraums 12 ist mit einer dünnen Schicht des bioresorbierbaren Materials des Knochennagels 1 verschlossen. Vorzugsweise wird nach dem Einfüllen des röntgenologisch darstellbaren Werkstoffs in die Öffnung 14 im balligen Kopf 9 des Knochennagels 1 ein kurzer zylindrischer Stopfen 15 eingeführt, der über die Oberfläche des Kopfes 9 übersteht. In einem weiteren Herstellungsschritt wird der überstehende Stopfen 15 erhitzt, so daß der bioresorbierbare Werkstoff schmilzt und das Material des Stopfens 15 sich über den balligen Kopf 9 verteilt und den mit dem Kontrastmittel 13 gefüllten Aufnahmeraum 12 sicher und dauerhaft verschweißt. Vorteilhafterweise ist die nach dem Schweißvorgang verbleibende Länge des in der Öffnung 14 verschweißten Stopfens 15 ungefähr gleich zu der Materialdicke des den Aufnahmeraum 12 umgebenden Schaftes 2.

Die Verwendung von festem Bariumsulfat, das vorzugsweise in Pulverform vorliegt, gestattet eine lange Standzeit des eingefüllten Kontrastmittels 15. Die dann vorliegende körnige Struktur des Röntgenkontrastmittels 15 ist widerstandsfähiger gegenüber der Freisetzung des Kontrastmittels 15.

Liegt das Röntgenkontrastmittel 15 dagegen in flüssiger Form vor, so kann es durch Poren in dem sich auflösenden bioresorbierbaren Schaft 2 schon vor dessen vollständiger Auflösung aus dem Aufnahmeraum 12 herausdiffundieren. Die Auswahl der Größe des Aufnahmeraums 12 bzw. der Konzentration des Röntgenkontrastmittels 15 in einem Befestigungsmittel 1 bestimmt die nachweisbare Zeitdauer der Resorption. Dabei wird diese Zeitdauer insbesondere durch die Dicke der Außenwände des Aufnahmeraums 12 festgelegt. Durch Röntgenaufnahmen der behandelten Körperpartie des Patienten kann in einfacher Weise aus dem jeweilig vorhandenen Kontrast des verbliebenen Röntgenkontrastmittels 15 auf die Auflösung des Befestigungsmittels 1 in der Knochensubstanz und damit den Heilungsfortschritt geschlossen werden.

Insbesondere kann als Röntgenkontrastmittel 15 in wässriger Lösung vorliegendes N,N′-Bis[2-hydroxy-1-(hydroxymethyl) etyhl]-2,4,6-triiod-5-lactamidoisophtalamid verwendet werden, das auch kurz mit Iopamidol (L-Form) bezeichnet wird.

Die an dem einführtiefen oder vorderseitigen Ende 6 des Knochennagels 1 vorgesehene Spitze 16 weist die Form eines Kegels mit einem spitzen Winkel von 90 Grad auf.

Die Verwendung eines 90-Grad-Kegels gestattet eine leichte Einführung des Knochennagels 1 in einen vorgebohrten Aufnahmeraum im Knochen. Sollte sich bei der Vorbohrung zur Erstellung des Loches für den Knochennagel 1 herausstellen, daß eine kürzerer Knochennagel 1 verwendet werden muß, so kann der Knochennagel 1 aus bioresorbierbarem Material durch Abschneiden eines Teils des unteren Bereichs 4 leicht auf die erforderliche Länge gekürzt werden.

Der beschriebene Knochennagel 1 weist einen Auszugswert von 600 Newton auf und ist auf Torsion und Scherung gut belastbar. Der Knochennagel wird derart eingeschlagen, daß der Kopf 9 gerade in der obersten Knorpelschicht verankert ist. Der obere glatte Bereich 3 des Schaftes 2 verläuft in der Corticalis, während der untere Bereich 4 sich mit seinen als Verankerungsstufen wirkenden Rippen 5 in die Spongiosa erstreckt.

Es können auch mehrere, verschieden große, mit dem Kontrastmittel 13 gefüllte Hohlräume 12 in das Befestigungsmittel entlang der Längsachse 8 eingebracht sein.

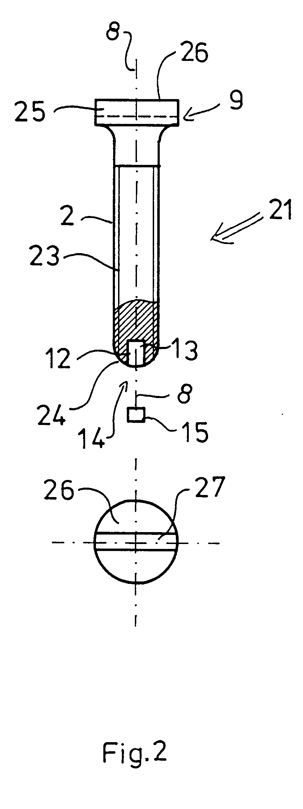

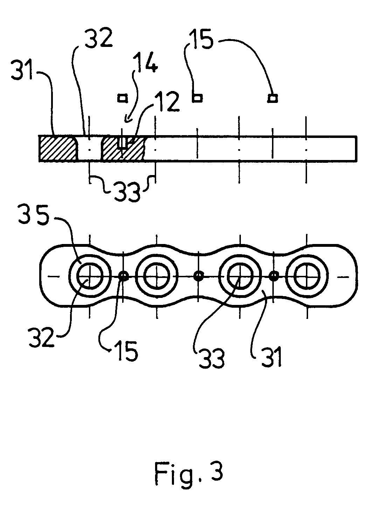

Der beschriebene mit dem Kontrastmittel 13 gefüllte Aufnahmeraum 12 kann ebenfalls in einem Kopf einer Knochenschraube aus bioresorbierbarem Material oder in einer Knochenplatte aus bioresorbierbarem Material vorgesehen sein, die in den Fig. 2 und 3 dargestellt sind.

Die Fig. 2 zeigt eine Seitenansicht und eine Draufsicht einer in einen Knochen eintreibbaren Knochenschraube 21. Die Knochenschraube 21 verfügt über einen zylindrischen, länglichen Schaft 2, der mit einem Gewinde 23 versehen ist und in einem abgerundeten Endbereich 24 ausläuft.

Die stiftförmige Knochenschraube 21 wird bei einer Operation durch das Knochenfragment des abgesplitterten Knochens und durch das Knochen-Fragmentlager in einem Bohrkanal in die Knochensubstanz eingeführt.

Der Kopf 9 der Knochenschraube 21 verfügt über eine zylindrische Verbreiterung 25 des Schaftes 2, wobei der Durchmesser des Kopfes 9 zwischen 10 und 50 Prozent größer als der Durchmesser des Schaftes 2 ist. Die rückseitige Endfläche 26 der Verbreiterung 25 des Kopfes 9 ist flach ausgeformt. In der Draufsicht auf die Knochenschraube 21 ist deutlich zu erkennen, daß in der Endfläche 26 ein Schlitz 27 zum Einsetzen eines Schraubendrehers vorgesehen ist.

Entlang der Achse 8 der Knochenschraube 21 ist in dem abgerundeten Endbereich 24 ein sich bis in den Schaft 2 erstreckender zylindrischer Aufnahmeraum 12 vorgesehen, der einen Durchmesser von zum Beispiel 0,6 Millimeter und eine Länge von vorzugsweise mindestens 0,5 Millimetern aufweist. In den Aufnahmeraum 12 ist das röntgenologisch darstellbare Kontrastmittel 13 eingefüllt.

Die an der rückseitigen Endfläche vorhandene Öffnung 14 des Aufnahmeraums 12 ist mit einer dünnen Schicht des bioresorbierbaren Materials der Knochenschraube 21 verschlossen. Vorzugsweise wird nach dem Einfüllen des röntgenologisch darstellbaren Werkstoffs in die Öffnung 14 der Knochenschraube 21 ein kurzer zylindrischer Stopfen 15 eingeführt, der über die Endfläche 24 übersteht. In einem weiteren Herstellungsschritt wird der überstehende Stopfen 15 erhitzt, so daß der bioresorbierbare Werkstoff schmilzt und das Material des Stopfens 15 sich über die Endfläche 24 verteilt und den mit dem Kontrastmittel 13 gefüllten Aufnahmeraum 12 sicher und dauerhaft verschweißt.

Die Fig. 3 zeigt eine Seitenansicht und eine Draufsicht einer Knochenplatte 31. Die gleichmäßig dicke Knochenplatte 31 verfügt über eine Viezahl von zylindrischen Befestigungslöchern 32, deren Längsachsen 33 rechtwinklig auf der Hauptebene der Knochenplatte 31 stehen. Durch die Befestigungslöcher 32 hindurch können z.B. Knochenschrauben 21 oder Knochenstifte 1 hindurchgeführt werden, um in einem Knochen verankert zu werden.

In der Draufsicht auf die Knochenplatte 31 sind deutlich die verbreiterten Absätze 35 zu erkennen, in denen jeweils ein Kopf 9 eines Knochenstiftes 1 oder einer Knochenschraube 21 ein Widerlager findet.

Parallel zu den Achsen 33 der Befestigungslöcher 32 in der Knochenplatte 31 sind zwischen diesen eine Vielzahl von zylindrischen Aufnahmeräumen 12 vorgesehen. In den Aufnahmeraum 12 ist das röntgenologisch darstellbare Kontrastmittel 13 eingefüllt.

Die jeweilige Öffnung 14 des Aufnahmeraums 12 ist mit einer dünnen Schicht des bioresorbierbaren Materials verschlossen. Vorzugsweise wird nach dem Einfüllen des röntgenologisch darstellbaren Werkstoffs in die Öffnungen 14 in der Knochenplatte 31 jeweils ein kurzer zylindrischer Stopfen 15 eingeführt. Neben den dargestellten Aufnahmeräumen 12 zwischen den Befestigungslöchern 32 sind natürlich auch Kontrastmitteldepots an den Endpunkten der Knochenplatte 13 oder in Rillen in der Oberfläche entlang der Knochenplatte 31 anordbar.

Das resorbierbare Material wird vorzugsweise aus der Gruppe der Polylactidsäuren, kurz PLA, oder der Polyglykolsäuren, kurz PGA, ausgewählt. Insbesondere Verbindungen aus Polylactidsäuren und Polyglykolsäuren sind geeignete bioresorbierbare Materialien, die auch als Lösungsmittel für das Röntgenkontrastmittel, insbesondere Bariumsulfat dienen können. Auch können Polydioxanone, kurz PDS, und bioresorbierbare Polyesterverbindungen zur Herstellung bioresorbierbarer Befestigungsmittel benutzt werden.

## Patentansprüche

1. Befestigungsmittel (1,21,31) aus einem bioresorbierbarem Werkstoff zur Fixierung von Knochenfragmenten mit mindestens einem mit einem röntgenologisch darstellbaren Kontrastmittel (13) gefüllten Aufnahmeraum (12), wobei das Kontrastmittel (13) in einer durch die Art des Kontrastmittels (13), dessen Konzentration, der Art des bioresorbierbaren Werkstoffs und die Außenwanddicke des Aufnahmeraums vorbestimmten Zeitdauer langsam parallel zur Auflösung des Befestigungsmittels (1,21,31) durch die Aufnahmeraumwände hindurchdiffundierbar ist.

2. Befestigungsmittel nach Anspruch 1, dadurch gekennzeichnet, daß der Aufnahmeraum (12) eine in dem Befestigungsmittel (1,21,31) vorgesehene zylindrische Ausnehmung ist.

3. Befestigungsmittel nach Anspruch 2, dadurch gekennzeichnet, daß die bohrlochferne Öffnung (14) des Zylinders (12) durch Erhitzen des die Öffnung (14) umgebenden Materials verschlossen und verschweißt ist.

4. Befestigungsmittel nach Anspruch 2, dadurch gekennzeichnet, daß ein Stopfen (15) in der bohrlochfernen Öffnung (14) des Zylinders (12) vorgesehen ist, wobei die Öffnung (14) durch Erhitzen des Stopfens (15) verschweißt ist.

5. Befestigungsmittel nach einem der Ansprüche 1 bis 4, dadurch gekennzeichnet, daß das in dem Aufnahmeraum (12) vorhandene Kontrastmittel (13) in flüssiger Form vorliegt.

6. Befestigungsmittel nach einem der Ansprüche 1 bis 4, dadurch gekennzeichnet, daß das in dem Aufnahmeraum (12) vorhandene Kontrastmittel (13) pulverförmig ist.

7. Befestigungsmittel nach einem der vorstehenden Ansprüche, dadurch gekennzeichnet, daß das in dem Aufnahmeraum (12) vorhandene Kontrastmittel (13) Bariumsulfat ist.

8. Befestigungsmittel nach einem der Ansprüche 1 bis 6, dadurch gekennzeichnet, daß das in dem Aufnahmeraum (12) vorhandene Kontrastmittel (13) gelöstes Iopamidol (L-Form) ist.

9. Befestigungsmittel nach einem der vorstehenden Ansprüche, dadurch gekennzeichnet, daß das Befestigungsmittel (1,21,31) ein in einen Knochen eintreibbarer Knochennagel (1) ist.

10. Befestigungsmittel nach Anspruch 9, dadurch gekennzeichnet, daß der Knochennagel (1) über einen Schaft (2) verfügt, in dessen vorderem einführtiefem Bereich (4) mehrere, umfänglich verlaufende Rippen (5) vorgesehen sind.

11. Befestigungsmittel nach Anspruch 10, dadurch gekennzeichnet, daß der Durchmesser der Rippen (5) um 5 bis 25 Prozent größer als der Durchmesser des Schaftes (2) ist.

12. Befestigungsmittel nach einem der Ansprüche 10 oder 11, dadurch gekennzeichnet, daß die über den Schaft (2) überstehenden Rippen (5) im Querschnitt die Form eines Dreiecks aufweisen, wobei die dem einführtiefen Ende (6) des Knochennagels (1) zugewandte Seite des Dreiecks mit der Achse des Schaftes (2) einen spitzen Winkel im Bereich zwischen 30 und 60 Grad bildet.

13. Befestigungsmittel nach Anspruch 12, dadurch gekennzeichnet, daß der Wert des spitzen Winkel 45 Grad beträgt.

14. Befestigungsmittel nach einem der Ansprüche 12 oder 13, dadurch gekennzeichnet, daß die dem Kopf (9) des Knochennagels (1) zugewandte Seite des Dreiecks mit der Achse des Schaftes (2) einen rechten Winkel bildet.

15. Befestigungsmittel nach einem der Ansprüche 9 bis 14, dadurch gekennzeichnet, daß der Kopf (9) des Knochennagels (1) eine konische Verbreiterung (11) des Schaftes (2) aufweist, wobei der größte Durchmesser des Kopfes (9) zwischen 10 und 50 Prozent größer als der Durchmesser des Schaftes (2) ist.

16. Befestigungsmittel nach Anspruch 15, dadurch gekennzeichnet, daß das kopfseitige Ende der konischen Verbreiterung (11) des Kopfes (9) des Knochennagels (1) ballig ausgeformt ist.

17. Befestigungsmittel nach einem der Ansprüche 1 bis 8, dadurch gekennzeichnet, daß das Befestigungsmittel (1,21,31) eine in einen Knochen eintreibbare Knochenschraube (21) ist.

18. Befestigungsmittel nach einem der Ansprüche 9 bis 17, dadurch gekennzeichnet, daß der Aufnahmeraum (12) in der Spitze des Befestigungsmittels (1,21) angeordnet ist.

19. Befestigungsmittel nach einem der Ansprüche 1 bis 8, dadurch gekennzeichnet, daß das Befestigungsmittel (1,21,31) eine Knochenplatte (31) ist, in der eine Vielzahl von mit Kontrastmittel (13) gefüllten und verschlossenen Aufnahmeräumen (12) angeordnet sind.

## Claims

1. Securing means (1, 21, 31) made of a bioabsorbable material and used for fixing bone fragments, having at least one receiver chamber (12) filled with a radiographic contrast medium (13), the contrast medium (13) being capable of diffusing slowly through the receiver chamber walls, in parallel with the dissolution of the securing means (1, 21, 31), in a period of time predetermined by the nature of the contrast medium (13), its concentration, the nature of the bioabsorbable material and the outer wall thickness of the receiver chamber.

2. Securing means according to Claim 1, characterized in that the receiver chamber (12) is a cylindrical recess provided in the securing means (1, 21, 31).

3. Securing means according to Claim 2, characterized in that the opening (14) of the cylinder (12) remote from the bore hole is sealed and welded by heating the material surrounding the opening (14).

4. Securing means according to Claim 2, characterized in that a stopper (15) is provided in the opening (14) of the cylinder (12) remote from the bore hole, the opening (14) being welded by heating the stopper (15).

5. Securing means according to one of Claims 1 to 4, characterized in that the contrast medium (13) present in the receiver chamber (12) is in liquid form.

6. Securing means according to one of Claims 1 to 4, characterized in that the contrast medium (13) present in the receiver chamber (12) is in powder form.

7. Securing means according to one of the preceding claims, characterized in that the contrast medium (13) present in the receiver chamber (12) is barium sulphate.

8. Securing means according to one of Claims 1 to 6, characterized in that the contrast medium (13) present in the receiver chamber (12) is dissolved iopamidol (L-form).

9. Securing means according to one of the preceding claims, characterized in that the securing means (1, 21, 31) is a bone nail (1) which can be driven into a bone.

10. Securing means according to Claim 9, characterized in that the bone nail (1) has a shaft (2) in whose front penetrating area (4) a plurality of ribs (5) are provided extending round the circumference.

11. Securing means according to Claim 10, characterized in that the diameter of the ribs (5) is 5 to 25 percent greater than the diameter of the shaft (2).

12. Securing means according to either of Claims 10 or 11, characterized in that the ribs (5) projecting from the shaft (2) have a triangular configuration in crosssection, the side of the triangle facing the penetrating end (6) of the bone nail (1) forming with the axis of the shaft (2) an acute angle in the range between 30 and 60 degrees.

13. Securing means according to Claim 12, characterized in that the value of the acute angle is 45 degrees.

14. Securing means according to either of Claims 12 or 13, characterized in that the side of the triangle facing the head (9) of the bone nail (1) forms a right angle with the axis of the shaft (2).

15. Securing means according to one of Claims 9 to 14, characterized in that the head (9) of the bone nail (1) has a conical widening (11) of the shaft (2), the maximum diameter of the head (9) being between 10 and 50 percent greater than the diameter of the shaft (2).

16. Securing means according to Claim 15, characterized in that the head-side end of the conical widening (11) of the head (9) of the bone nail (1) is of a crowned shape.

17. Securing means according to one of Claims 1 to 8, characterized in that the securing means (1, 21, 31) is a bone screw (21) which can be driven into a bone.

18. Securing means according to one of Claims 9 to 17, characterized in that the receiver chamber (12) is arranged in the tip of the securing means (1, 21).

19. Securing means according to one of Claims 1 to 8, characterized in that the securing means (1, 21, 31) is a bone plate (31) in which are arranged a plurality of receiver chambers (12) filled with contrast medium (13) and sealed.

## Revendications

1. Moyen de fixation (1, 21, 31) en matière biorésorbable pour fixer des fragments osseux ayant au moins un logement (12) rempli d'une substance de contraste visible à la radiographie (13), dans lequel la substance de contraste (13) est susceptible de diffuser lentement à travers les parois du logement au fur et à mesure de la dissolution du moyen de fixation (1, 21, 31), en un temps déterminé par le type de substance de contraste, par sa concentration, et en fonction de la matière biorésorbable et de l'épaisseur de la paroi externe du logement.

2. Moyen de fixation selon la revendication 1, caractérisé en ce que le logement (12) est un évidement cylindrique prévu dans le moyen de fixation (1, 21, 31).

3. Moyen de fixation selon la revendication 2, caractérisé en ce que l'ouverture (14) éloignée du perçage du cylindre (12) est obturée et soudée par chauffage de la matière entourant l'ouverture (14).

4. Moyen de fixation selon la revendication 2, caractérisé en ce qu'un bouchon (15) est prévu dans l'ouverture (14) éloignée du perçage du cylindre (12), l'ouverture (14) étant soudée par chauffage du bouchon (15).

5. Moyen de fixation selon l'une ou l'autre des revendications 1 à 4, caractérisé en ce que la substance de contraste (13) placée dans le logement (12) se présente sous forme liquide.

6. Moyen de fixation selon l'une ou l'autre des revendications 1 à 4, caractérisé en ce que la substance de contraste (13) placée dans le logement (12) se présente sous forme de poudre.

7. Moyen de fixation selon l'une ou l'autre des revendications précédentes, caractérisé en ce que la substance de contraste (13) placée dans le logement (12) est du sulfate de baryum.

8. Moyen de fixation selon l'une ou l'autre des revendications 1 à 6, caractérisé en ce que la substance de contraste (13) placée dans le logement (12) est une solution d'Iopamidol (forme L).

9. Moyen de fixation selon l'une ou l'autre des revendications précédentes, caractérisé en ce que le moyen de fixation (1, 21, 31) est un clou orthopédique (1) prévu pour être placé dans un os.

10. Moyen de fixation selon la revendication 9, caractérisé en ce que le clou orthopédique (1) se compose d'une tige allongée (2), dont la partie avant (4), servant à l'introduction, est munie de nervures périphériques (5).

11. Moyen de fixation selon la revendication 10, caractérisé en ce que le diamètre des nervures (5) est supérieur de 5 à 25 % au diamètre de la tige (2).

12. Moyen de fixation selon l'une ou l'autre des revendications 10 et 11, caractérisé en ce que, vues en coupe, les nervures (5) disposées sur la tige (2) sont en forme de triangle, le côté du triangle qui est dirigé vers l'extrémité d'introduction (6) du clou orthopédique (1) forme avec l'axe de la tige (2) un angle aigu d'environ 30 à 60 degrés.

13. Moyen de fixation selon la revendication 12, caractérisé en ce que la valeur de l'angle aigu est de 45 degrés.

14. Moyen de fixation selon l'une ou l'autre des revendications 12 et 13, caractérisé en ce que le côté du triangle, qui est dirigé vers la tête (9) du clou orthopédique (1), forme avec l'axe de la tige (2), un angle droit.

15. Moyen de fixation selon l'une ou l'autre des revendications 9 à 14, caractérisé en ce que la tête (9) du clou orthopédique (1) présente un prolongement conique (11) de la tige (2), le plus grand diamètre de la tête (9) étant supérieur de 10 à 50 % au diamètre de la tige (2).

16. Moyen de fixation selon la revendication 15, caractérisé en ce que la face terminale du prolongement conique (11) de la tête (9) du clou orthopédique (1) a une forme bombée.

17. Moyen de fixation selon l'une ou l'autre des revendications 1 à 8, caractérisé en ce que le moyen de fixation (1, 21, 31) est un clou orthopédique (21) prévu pour être placé dans un os.

18. Moyen de fixation selon l'une ou l'autre des revendications 9 à 17, caractérisé en ce que le logement (12) est prévu à la pointe du moyen de fixation (1, 21).

19. Moyen de fixation selon l'une ou l'autre des revendications 1 à 8, caractérisé en ce que le moyen de fixation (1, 21, 31) est une plaque orthopédique (31) dans laquelle sont prévus plusieurs logements (12) remplis de substance de contraste (13) et obturés.
